# EUROPÄISCHE PATENTSCHRIFT

(11) **EP 0 902 642 B1**
(45) Veröffentlichungstag und Bekanntmachung des Hinweises auf die Patenterteilung: **29.06.2005**
(21) Anmeldenummer: 96909974.6
(22) Anmeldetag: 23.04.1996
(51) Int. Cl.: A61B 5/00, A61B 6/14, A61C 13/00

(54) **DIAGNOSTIKEINRICHTUNG MIT EINER MOBILEN SIGNALAUFNAHMEEINRICHTUNG UND EINER STATIONÄREN AUSWERTEEINRICHTUNG**
DIAGNOSTIC DEVICE WITH A MOBILE SIGNAL RECORDING DEVICE AND A STATIONARY EVALUATION DEVICE
SYSTEME DIAGNOSTIQUE COMPORTANT UN DISPOSITIF MOBILE D'ENREGISTREMENT DE SIGNAUX ET UN DISPOSITIF STATIONNAIRE D'EVALUATION

(30) Priorität: 04.05.1995 DE 19516451
(43) Veröffentlichungstag der Anmeldung: 24.03.1999
(73) Patentinhaber: Sirona Dental Systems GmbH, 64625 Bensheim (DE)
(72) Erfinder: REINKE, Michael, D-64289 Darmstadt (DE); SCHULZE-GANZLIN, Ulrich, D-64653 Lorsch (DE)
(74) Vertreter: Sommer, Peter
(86) Internationale Anmeldenummer: PCT/DE1996/000700
(87) Internationale Veröffentlichungsnummer: WO 1996/034556

(56) Entgegenhaltungen:
- EP-A- 0 299 490
- EP-A- 0 544 974
- WO-A-91/16850
- WO-A-94/13198
- WO-A-96/03917
- US-A- 4 935 635
- US-A- 5 434 418

## Beschreibung

Es sind Röntgendiagnostikeinrichtungen bekannt, die eine Aufnahmeeinheit aus Strahlensender und Strahlenempfänger sowie eine damit in Verbindung stehende stationäre Auswerteeinrichtung aufweisen. Hierbei werden die beim Durchstrahlen eines Objekts gewonnenen elektrischen Signale über Kabel der stationären Auswerteeinrichtung zugeführt. Die stationäre Auswerteeinrichtung wandelt diese Signale in Bildsignale, die dann auf einem Monitor als Bild des Objekts darstellbar sind.

Eine derartige Einrichtung ist aus EP-A-0 544 974 bekannt. Die US 4 936 635 A1 beschreibt ein System zur Vermessung von Objekten in drei Dimensionen. In einer bevorzugten Ausführungsform wird dieses System zur 3D-Vermessung einer Mundhöhle verwendet. Das System besteht aus einer stationären Auswerteeinheit und einer mobilen Erfassungseinheit, die mittels Triangulation Punktdatensätze erstellt und die der stationären Auswerteeinheit auf verschiedenen Wegen zugeführt werden, so zum Beispiel Disketten, Glasfaseroptik, LAN-Verbindungen (Local Area Network) oder andere Kommunikationssysteme mit hoher Bandbreite, wo sie ausgewertet und gegebenenfalls weiterverarbeitet werden.

Aufgabe der Erfindung ist die weitere vorteilhafte Ausgestaltung einer Diagnostikeinrichtung, insbesondere soll diese so ausgeführt werden, daß eine kostengünstige Handhabung bei hohem Nutzungskomfort gewährleistet ist.

Die Aufgabe wird erfindungsgemäß gelöst durch eine Diagnostikeinrichtung mit einer mobilen Signalaufnahmeeinrichtung, die einen Strahlenempfänger zum Erzeugen elektrischer Signale in Abhängigkeit vom Strahlenschatten eines durchstrahlten Objekts,
die eine Rechen- und Speichereinheit, eine Anzeige- sowie eine Kommunikationseinrichtung aufweist und
mit einer eine Kommunikationseinrichtung aufweisenden stationären Auswerteeinrichtung,
wobei die Kommunikationseinrichtung als bidirektionale Kommunikationseinrichtung ausgeführt ist und zur Signalübertragung
zwischen der mobilen Signalaufnahmeeinrichtung und der stationären Auswerteeinrichtung dient.

Vorteil der Erfindung ist, daß über die mobile Signalaufnahmeeinrichtung die vom Strahlenempfänger erzeugten elektrischen Signale aufgenommen, gegebenenfalls gespeichert und durch bidirektionale Kommunikation der stationären Auswerteeinrichtung zugeführt oder von der stationären Auswerteeinrichtung abgefragt werden können.

Die mobile Signalaufnahmeeinrichtung kann vorteilhafterweise in mehreren Untersuchungsräumen eingesetzt werden, so daß nur eine mobile Signalaufnahmeeinrichtung benötigt wird. Die Signalübertragung erfolgt drahtlos. Sind mehrere Untersuchungsräume vorgesehen, so kann es auch vorteilhaft sein, wenn weitere stationäre Auswerteeinrichtungen und gegebenenfalls auch weitere mobile Signalaufnahmeeinrichtungen vorgesehen sind, die über ein Netzwerk miteinander in Verbindung stehen. Die Auswertung der von den mobilen Signalaufnahmeeinrichtungen gewonnenen Signale kann somit von einer der stationären Auswerteeinrichtungen vorgenommen werden.

Weitere Vorteile und Einzelheiten der Erfindung ergeben sich aus der nachfolgenden Beschreibung eines Ausführungsbeispiels anhand der Zeichnungen in Verbindung mit den Unteransprüchen.

Es zeigt:
Figur 1 eine Röntgendiagnostikeinrichtung nach der Erfindung,
Figur 2 eine Struktur einer mobilen Signalaufnahmeeinrichtung der Röntgendiagnostikeinrichtung nach Figur 1,
Figur 3 eine Netzwerkprogrammierschnittstelle der Röntgendiagnostikeinrichtung nach Figur 1 und
Figur 4 ein Schema eines Aufnahmeablaufs der Röntgendiagnostikeinrichtung nach Figur 1.

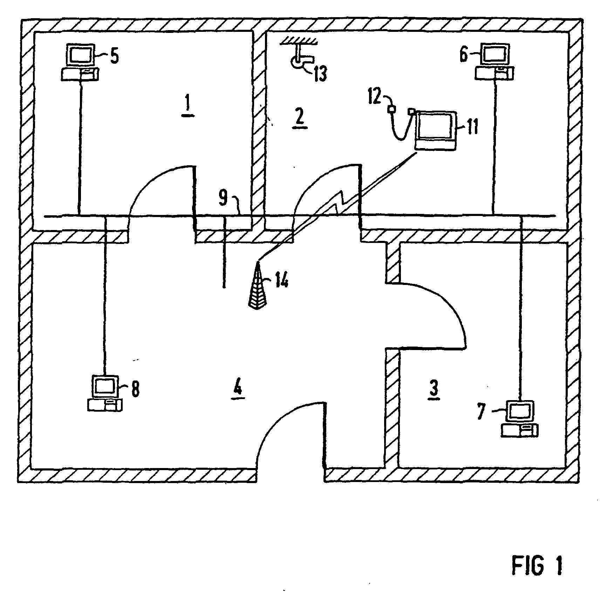

In der Figur 1 ist ein Ausführungsbeispiel einer Röntgendiagnostikeinrichtung nach der Erfindung gezeigt. Diese Röntgendiagnostikeinrichtung weist unterschiedliche Räume 1, 2, 3, 4 auf, in denen Computer, beispielsweise Personal-Computer 5, 6, 7, 8, als stationäre Auswerteeinrichtungen vorgesehen sind, die über ein Datennetzwerk 9 miteinander in Verbindung stehen. In einem Raum 2 ist eine mobile Signalaufnahmeeinrichtung 11 vorhanden. Diese mobile Signalaufnahmeeinrichtung 11 besitzt einen Strahlenempfänger 12, der den beim Durchstrahlen eines Untersuchungsobjekts durch Strahlung eines Strahlensenders 13 erzeugten Strahlenschatten in elektrische Signale wandelt. Die mobile Signalaufnahmeeinrichtung 11 steht über eine Basisstation 14 mit dem Netzwerk 9 und damit mit den Computern 5, 6, 7, 8 in einer bidirektionalen Verbindung. Besonders bevorzugt besteht zwischen der mobilen Signalaufnahmeeinrichtung 11 und der Basisstation 14 eine bidirektionale Funkverbindung. Alternativ kann eine Infrarotverbindung vorgesehen sein.

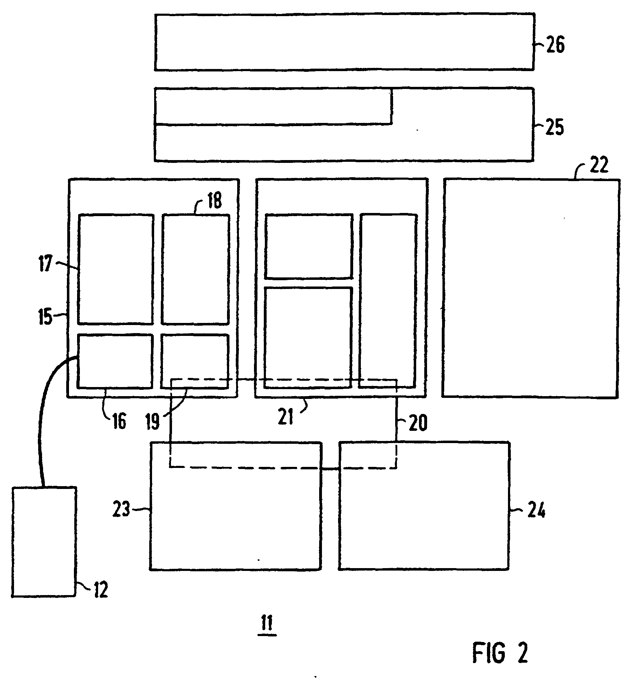

Mit Verweis auf die Figur 2 soll nunmehr ein Ausführungsbeispiel einer mobilen Signalaufnahmeeinrichtung 11 näher erläutert werden. Diese mobile Signalaufnahmeeinrichtung 11 besitzt, wie bereits erläutert, einen Strahlenempfänger 12, dessen Signale einem Bilderfassungssubsystem 15 zugeführt werden, das einen Analogteil 16, einen Strahlungserkennungsteil 17, einen lokalen Bildspeicher 18 sowie einen DMA-Teil 19 aufweist. Im Analogteil 16 werden die vom Strahlenempfänger 12 ausgehenden analogen Signale mittels eines Analog/Digital-Wandlers in digitale Signale gewandelt. Diese digitalen Signale werden in einem Speicher (RAM - Random Memory) abgelegt, was besonders schnell erfolgen kann, wenn hierzu ein Subsystem (DMA) verwendet wird, das einen direkten Zugriff zu diesem Speicher hat. Das Bilderfassungssubsystem ist über einen Bus 20 mit einem lokalen Rechner 21, einer Anzeige 22, einer Netzwerkkarte 23 sowie weiteren Zusatzkarten 24 verbunden.

Der Strahlenempfänger 12 kann hierbei vorzugsweise in drei Phasen betrieben werden:
1. Bereitschaft (beliebig lang), auftreffende Strahlung wird detektiert.
2. Nach erfolgter Strahlendetektion erfolgt schnellstmögliche Umschaltung in die Integrationsphase, in der das Röntgenschattenbild vom Strahlenempfänger 12 in ein zweidimensionales Ladungsbild gewandelt wird.
3. Austakten des Ladungsbildes in das Bilderfassungssubsystem mit anschließender Digitalisierung und Übertragung zu dem Computer 5, 6, 7, 8.

Zur Spannungsversorgung der mobilen Signalaufnahmeeinrichtung 11 kann ein austauschbarer Akku 25 vorgesehen sein, der von einer stationären Ladestation 26 mit Energie versorgt werden kann. Die Ladestation 26 kann hierbei als stationäre Tisch- oder Wandhalterung dienen, die eine problemlose und schnelle Handhabung ermöglicht. Damit die mobile Signalaufnahmeeinrichtung 11 klein dimensioniert und kostengünstig hergestellt werden kann und einen geringen Stromverbrauch aufweist, ist es vorteilhaft, wenn keine Möglichkeit zur Bilddarstellung oder zum Patientendialog vorgesehen ist. Der Benutzerdialog erfolgt über den bzw. die Computer 5, 6, 7, 8, wobei die mobile Signalaufnahmeeinrichtung 11 Status- und Fehlermeldungen mittels einer alphanumerischen Anzeige oder über LEDs anzeigt. Gleichwohl ist auch eine Bilddarstellung mittels Flachbildschirm, z.B. LCD, sinnvoll.

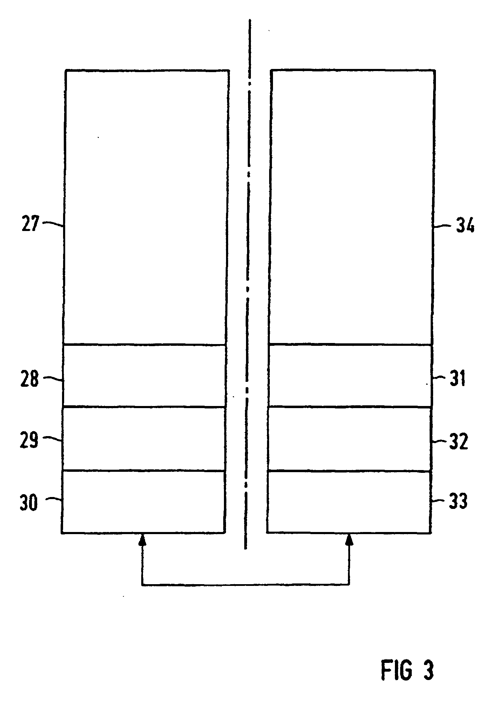

In der Figur 3 ist ein Blockschaltbild einer Netzwerkprogrammierschnittstelle zwischen der mobilden Signalaufnahmeeinrichtung 11 und dem Computer 5, 6, 7, 8 dargestellt. Die mobile Signalaufnahmeeinrichtung 11 umfaßt einen ersten Block 27, nämlich der Bilderzeugung, des Bildtransfers und der Kommunikation zwischen der mobilen Signalaufnahmeeinrichtung und dem Computer 5, 6, 7, 8. Die Bilderzeugung umfaßt, wie bereits erläutert, die drei Betriebsphasen:
1. Bereitschaft,
2. Strahlendetektion und
3. Austakten der Signale des Strahlenempfängers 12 über den Analog/Digital-Wandler und den DMA und Eintakten in den RAM der mobilen Signalaufnahmeeinrichtung 11.

Der Bildtransfer, d.h. die Übertragung der dem empfangenen Strahlenschatten entsprechenden Signale, erfolgt aus dem RAM über eine Netzwerkkarte zum Computer 5, 6, 7, 8. Die Kommunikation zwischen der mobilen Signalaufnahmeeinrichtung 11 und dem Computer 5, 6, 7, 8 erfolgt hierbei über ein bidirektionales Senden, nicht nur von Bilddaten, sondern auch zu Kontrollzwecken, Fehlermeldungen und Statusanzeigen. Es ist ein weiterer Block 28 betreffend das Netzwerk API (Application Programming Interface), ein Block 29 betreffend das Betriebssystem und ein Block 30 betreffend die Netzwerkschicht vorgesehen. Zu den Blöcken 28 bis 30 korrespondierende Blöcke enthält auch das Blockschaltbild des Computers 5, 6, 7, 8 beispielsweise eines Servers 10 und zusätzlich einen Block 34 zur Bildverarbeitung der Bildsignale des Strahlenempfängers 12 zur Patientenauswahl und Patientenzuordnung sowie der Bildarchivierung. Zur Datenübertragung zwischen den Computern 5, 6, 7, 8, und auch zwischen dem Computer der mobilen Signalaufnahmeeinrichtung 11 und den Computern 5, 6, 7, 8 sind mehrere Schritte erforderlich, die in sogenannten Schichten (Layer) passiert werden. Diese Schichten sind teilweise standardisiert und es existieren dazu entsprechende Software-Protokolle. Diese Software muß sowohl auf der mobilen Signalaufnahmeeinrichtung 11 als auch auf dem Computer 5, 6, 7, 8 vorhanden sein, damit die zu übertragenden Informationen (Bilddaten, Status) zwischen den Blöcken 27 und 34 ausgetauscht werden können.

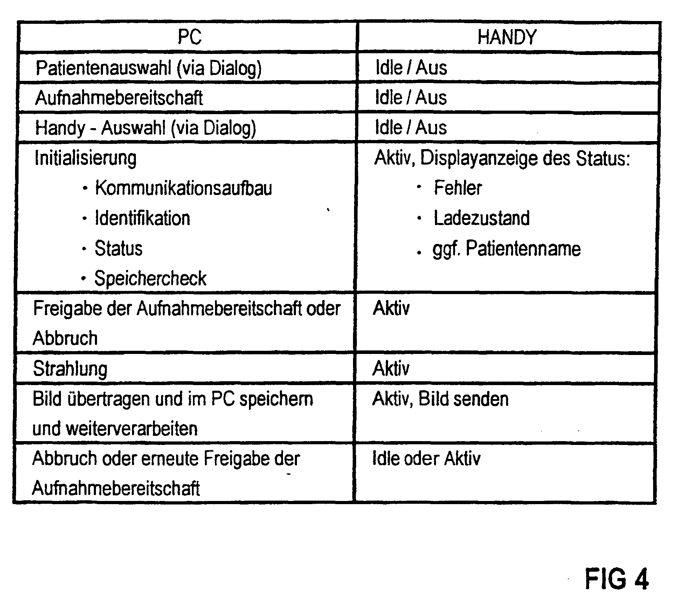

Ein Ausführungsbeispiel eines Ablaufs zur Erstellung einer Röntgenaufnahme in einem bidirektionalen Kommunikationssystem ist beispielsweise in der Figur 4 dargestellt und bezieht sich insbesondere auf die Kommunikation über ein Funk-Netzwerk (Funk-LAN).

Als Software-Struktur hat sich hierbei eine verteilte Client-Server-Lösung als besonders vorteilhaft erwiesen. Hierdurch kann eine beliebige Anzahl mobiler Signalaufnahmeeinrichtungen 11 über das Netzwerk mit einer ebenfalls beliebigen Anzahl stationärer Auswerteeinrichtungen kommunizieren. Die Aufgabe des Clients ist die Erfassung der Rohbilddaten und die Weiterreichung dieser Daten an einen der Computer 5, 6, 7, 8. Dieser hat die Aufgabe, diese Rohdaten weiterzuverarbeiten und patientenbezogen zu archivieren.

Im Rahmen der Erfindung kann der Strahlenempfänger 12 nicht nur zum Wandeln eines Röntgenstrahlenschattens sondern eine Einrichtung zur Messung eines 3-D-Bildes zur Zahnrestauration (CEREC), eine intraorale Farbvideokamera zur Diagnose, eine Einrichtung zur Messung der Zahntaschentiefe, eine Einrichtung zur Messung der Zahnfestigkeit im Kiefer (PERIOTEST), eine Einrichtung zur Messung und Überprüfung der Occlusion und/oder eine Einrichtung zur Messung chemischer Daten (pH-Wert) des Speichels sein. Es ist selbstverständlich, daß die Einrichtungen dann jeweils ein entsprechendes Steuerungs- und Erfassungssystem aufweisen.

Bei der bidirektionalen Kommunikation soll sichergestellt werden, daß die Signale sicher von der mobilen Signalaufnahmeeinrichtung 11 zum Computer 5, 6, 7, 8 und in einer Form übertragen werden, daß der korrekte Empfang der Bilddaten quittiert und im Fehlerfall gegebenenfalls wiederholt wird. Dieser wichtige Sicherheitsaspekt dient zur Strahlenhygiene.

## Patentansprüche

1. Diagnostikeinrichtung für den Dentalbereich, die mindestens eine Auswerteeinrichtung (5, 6, 7, 8) und eine mobile Signalaufnahmeeinrichtung (11) umfasst, welche eine Rechen- und Speichereinheit (21) sowie eine Kommunikationseinrichtung (23) enthält, **dadurch gekennzeichnet, dass** die Kommunikationseinrichtung (23) der Signalaufnahmeeinrichtung (11) als bidirektionale Kommunikationseinrichtung zur drahtlosen Signalübertragung ausgeführt ist, wobei die gegebenenfalls in mehreren Diagnoseräumen (1, 2, 3, 4) angeordneten stationären Auswerteeinrichtungen (5, 6, 7, 8) Kommunikationseinrichtungen enthalten, die als bidirektionale Kommunikationseinrichtungen ausgeführt sind und zur drahtlosen Signalübertragung zwischen der mobilen Signalaufnahmeeinrichtung (11) und den stationären Auswerteeinrichtungen (5, 6, 7, 8) dienen.

2. Diagnostikeinrichtung nach Anspruch 1, wobei mehrere stationäre Auswerteeinrichtungen und mehrere mobile Signalaufnahmeeinrichtungen (11) vernetzt sind.

3. Diagnostikeinrichtung nach einem der Ansprüche 1 oder 2 zur zahnmedizinischen Diagnose mittels Röntgenstrahlung.

4. Diagnostikeinrichtung nach Anspruch 1 oder 2, **gekennzeichnet durch** eine Einrichtung zur Messung eines 3-D-Bildes zur Zahnrestauration.

5. Diagnostikeinrichtung nach Anspruch 1 oder 2,
**gekennzeichnet durch** eine intraorale Farbvideokamera zum Erzeugen elektrischer Signale.

6. Diagnostikeinrichtung nach Anspruch 1 oder 2,
**gekennzeichnet durch** eine Einrichtung zur Messung der Zahntaschentiefe.

7. Diagnostikeinrichtung nach Anspruch 1 oder 2, **gekennzeichnet durch** eine Einrichtung zur Messung der Zahnfestigkeit im Kiefer.

8. Diagnostikeinrichtung nach Anspruch 1 oder 2,
**gekennzeichnet durch** eine Einrichtung zur Messung und Überprüfung der Occlusion.

9. Diagnostikeinrichtung nach Anspruch 1 oder 2,
**gekennzeichnet durch** eine Einrichtung zur Messung chemischer Daten des Speichels.

## Claims

1. A dental diagnostic system, comprising at least one evaluation system (5, 6, 7, 8) and at least one mobile signal recording system (11) containing a computing and storing unit (21) and a communication system (23), **characterized in that** said communication system (23) of said signal recording system (11) is in the form of a bidirectional wireless transmission system, whilst said stationary evaluation systems (5, 6, 7, 8) disposed in one or more diagnosis rooms (1, 2, 3, 4) contain communication systems that are in the form of bidirectional communication systems and serve to provide wireless transmission between said mobile signal recording system (11) and said stationary evaluation systems (5, 6, 7, 8).

2. A diagnostic system as defined in claim 1, wherein a plurality of stationary evaluation systems and a plurality of mobile signal recording systems (11) are networked.

3. A diagnostic system as defined in claim 1 or claim 2 for effecting dental diagnosis by means of X-ray radiation.

4. A diagnostic system as defined in claim 1 or claim 2, **characterized by** means for acquisition of 3D-scans for tooth restorations.

5. A diagnostic system as defined in claim 1 or claim 2, **characterized by** an intraoral color video camera for generating electric signals.

6. A diagnostic system as defined in claim 1 or claim 2, **characterized by** means for measuring the depth of a dental pocket.

7. A diagnostic system as defined in claim 1 or claim 2, **characterized by** means for measuring the firmness of a tooth in its socket.

8. A diagnostic system as defined in claim 1 or claim 2, **characterized by** means for measuring and examining the occlusion.

9. A diagnostic system as defined in claim 1 or claim 2, **characterized by** means for measuring chemical data of the saliva.

## Revendications

1. Dispositif diagnostique pour le domaine dentaire, comprenant au moins un dispositif d'évaluation (5, 6, 7, 8) et un dispositif mobile d'enregistrement de signaux (11) qui contient une unité de calcul et de mémoire (21) ainsi qu'un dispositif de communication (23), **caractérisé en ce que** le dispositif de communication (23) du dispositif d'enregistrement de signaux (11) est réalisé comme un dispositif de communication bidirectionnelle pour une transmission de signaux sans fil, dans lequel les dispositifs stationnaires d'évaluation (5, 6, 7, 8), éventuellement disposés dans plusieurs salles de diagnostique (1, 2, 3, 4), comprennent des dispositifs de communication qui sont réalisés comme des dispositifs de communication bidirectionnelle et servent à la transmission de signaux sans fil entre le dispositif mobile d'enregistrement de signaux (11) et les dispositifs stationnaires d'évaluation (5, 6, 7, 8).

2. Dispositif diagnostique selon la revendication 1, dans lequel plusieurs dispositifs stationnaires d'évaluation et plusieurs dispositifs mobiles d'enregistrement de signaux (11) sont mis en réseau.

3. Dispositif diagnostique selon l'une quelconque des revendications 1 ou 2 pour un diagnostic en médecine dentaire au moyen d'un rayonnement X.

4. Dispositif diagnostique selon la revendication 1 ou 2, **caractérisé par** un dispositif permettant de mesurer une image 3D pour une restauration dentaire.

5. Dispositif diagnostique selon la revendication 1 ou 2, **caractérisé par** une caméra vidéo couleur intra-buccale permettant de produire des signaux électriques.

6. Dispositif diagnostique selon la revendication 1 ou 2, **caractérisé par** un dispositif permettant de mesurer la profondeur de poche parodontale.

7. Dispositif diagnostique selon la revendication 1 ou 2, **caractérisé par** un dispositif permettant de mesurer la stabilité dentaire dans la mâchoire.

8. Dispositif diagnostique selon la revendication 1 ou 2, **caractérisé par** un dispositif permettant de mesurer et de vérifier l'occlusion.

9. Dispositif diagnostique selon la revendication 1 ou 2, **caractérisé par** un dispositif permettant de mesurer des données chimiques de la salive.
